# EUROPEAN PATENT APPLICATION

(11) **EP 4 800 007 A1**
(43) Date of publication of application: **02.09.2026**
(21) Application number: 25160616.6
(22) Date of filing: 27.02.2025
(51) Int. Cl.: C07D 225/02, B01F 23/00, C07C 45/58, C07C 49/413, C07C 249/04, C07C 251/44, C07D 301/12

(54) **METHOD FOR EPOXIDIZING MONOETHYLENICALLY UNSATURATED ORGANIC COMPOUNDS**

(71) Applicant: Evonik Operations GmbH, 45128 Essen (DE)
(72) Inventor: VOGELSANG, Dennis, 48249 Dülmen (DE); LIENKE, Achim, 3124ND Schiedam (NL)
(74) Representative: Evonik Patent Association

(57) **Abstract**

The present invention relates to a method for epoxidizing a monoethylenically unsaturated organic compound. Moreover, the invention relates to an apparatus for carrying out the method and to a composition obtained in the method. The present invention further relates to a method for synthesizing a lactam.

## Description

The present invention relates to a method for epoxidizing a monoethylenically unsaturated organic compound. Moreover, the invention relates to an apparatus for carrying out the method and to a composition obtained in the method. The present invention further relates to a method for synthesizing a lactam.

The epoxidation of ethylenically unsaturated organic compounds using catalyst systems and peroxide oxidants plays a crucial role in industrial processes, in particular for the synthesis of lactams, and remains a key area of research for various applications. For example, the epoxidation of cyclododecene (CDEN) to epoxycyclododecane (CDAN-epoxide) plays a crucial role in the large-scale production of laurolactam. In this process, the formed CDAN-epoxide undergoes rearrangement to form cyclododecanone (CDON), which is subsequently converted into the corresponding oxime. Finally, laurolactam is obtained via Beckmann rearrangement. The laurolactam serves as a key precursor for the production of nylon-12.

The epoxidation of CDEN with hydrogen peroxide as oxidant in a biphasic mixture is, for example, described in WO 2018/002114 A1, EP 2 946 831 A2 and WO 2021/085978 A1. For this process, significant optimization efforts have already been made to improve yields and make the process more economical. One notable area of focus has been the recovery and reactivation of the used catalyst system, as described, for instance, in EP 2 946 831 A2 or WO 2018/002114 A1.

However, considering the entire cascade from CDEN to laurolactam, each step of the process produces waste streams which require extensive treatment to recover and recycle the by-products from these streams. In EP 2 980 071 A1 a reaction sequence including the epoxidation of CDEN and the subsequent rearrangement to CDON is described, which can be applied in the process for synthesizing laurolactam. It is described that during the rearrangement of the epoxide to CDON, a stream is obtained containing a mixture of by-products, such as CDAN, CDEN, cyclododecanol (CDOL) and high-boiling compounds. These by-products are separated, for example, by distillation, then converted into usable compounds through processes like hydrogenation or oxidation, and finally reintroduced into the main process of the laurolactam synthesis.

These procedures require several additional steps for which equipment, staff and time are needed. Thus, there remains a need to optimize the process for greater economic efficiency and more effective usage of waste streams and by-products.

Surprisingly, it has now been found that the epoxidation of monoethylenically unsaturated organic compounds can be carried out in the presence of streams containing primary alcohols, secondary alcohols or polyethylenically unsaturated organic compounds, all of which can be by-products of at least one of the reaction steps of the synthesis of laurolactam starting from CDEN. This allows the waste stream of a reaction step to be reused and by-products to be converted into either useful or easily separable compounds. Additional purification steps and conversion processes of waste streams and by-products can be eliminated, making the overall process more efficient.

Accordingly, the present invention relates to a method for epoxidizing a monoethylenically unsaturated organic compound, comprising the steps of
(i) mixing a first stream comprising the monoethylenically unsaturated organic compound with up to 25 wt.-% of a second stream comprising a primary alcohol, a secondary alcohol, a polyethylenically unsaturated organic compound or a combination of any of them, wherein wt.-% is based on the total weight of the combined first and second stream; and
(ii) oxidizing the mixture obtained in step (i) by at least one peroxide in the presence of a catalyst.

Moreover, the present invention relates to a method for synthesizing a lactam, comprising the steps of:
(i) epoxidizing a cyclic monoethylenically unsaturated organic compound to an epoxide;
(ii) rearranging the epoxide to a ketone;
(iii) converting the ketone to an oxime; and
(iv) rearranging the oxime to the lactam;
wherein step (i) comprises the epoxidation according to the method described herein.

Furthermore, the present invention relates to an apparatus for carrying out the epoxidation method described herein, wherein the apparatus comprises
(i) a first stirred tank reactor or a first series of stirred tank reactors coupled to each other, being adapted to carry out the oxidation of step (ii);
(ii) a second stirred tank reactor or a second series of stirred tank reactors coupled to each other, being adapted to carry out a further reaction of the oxidation product of step (ii) or a subsequent reaction product thereof, thereby producing a waste stream comprising a primary alcohol, a secondary alcohol, a polyethylenically unsaturated organic compound or a combination of any of them; and
(iii) a pipeline connecting the second stirred tank reactor or series of stirred tank reactors with the first stirred tank reactor or series of stirred tank reactors to direct the waste stream produced in the second stirred tank reactor or series of stirred tank reactors to the first stirred tank reactor or series of stirred tank reactors.

In addition, the present invention relates to a monophasic or biphasic composition comprising an organic phase, wherein the organic phase comprises an epoxidized monoethylenically unsaturated organic compound in an amount of from 50 to 99 wt.-%, preferably of from 75 to 98 wt.-%, a derivative of the monoethylenically unsaturated organic compound comprising a ketone functionality in an amount of from 1.0 to 20 wt.-%, preferably of from 2.0 to 20 wt.-%, and tungsten in an amount of from 500 to 5000 ppm, preferably of from 1000 to 3000 ppm; wherein wt.-% and ppm are based on the total weight of the organic phase.

As used herein, the term "comprising" and variations thereof are used synonymously with the terms "including", "containing" and variations thereof and are understood to be open and non-limiting terms which do not exclude the presence of additional undescribed or unrecited elements, compounds, ingredients or process steps. As used herein, the term "consisting of" is understood to exclude the presence of unspecified elements, compounds, ingredients or process steps. When the non-limiting terms "comprising", "including" or "containing" are used in the present specification, the case of "consisting of" is included therein. For example, a process described as "comprising" or "including" certain steps can consist of the explicitly recited steps or can further comprise one or more unrecited steps. Same applies, for example, to compositions and their corresponding explicitly described or non-recited ingredients.

In this application, the indefinite article "a" means one or more of what it denotes.

As stated above, the present invention relates to a method for epoxidizing a monoethylenically unsaturated organic compound. According to the present invention, the term "epoxidation" is to be understood in its usual meaning, namely, the oxidation of an ethylenically unsaturated organic compound with a peroxide to form an epoxide functional group (oxirane).

As used herein, the term "ethylenically unsaturated organic compound" refers to an organic compound having at least one carbon-carbon double bond and/or at least one carbon-carbon triple bond, preferably at least one carbon-carbon double bond. The term "monoethylenically unsaturated organic compound" refers to an organic compound having exactly one carbon-carbon double bond or one carbon-carbon triple bond, preferably one carbon-carbon double bond.

According to the present invention, for the monoethylenically unsaturated organic compound preference is given to an organic compound having a total of six to twenty carbon atoms, preferably a cyclic organic compound having a total of six to twenty carbon atoms, more preferably a cyclic monoethylenically unsaturated C12 compound, most preferably cyclododecene (CDEN). The CDEN may be obtained from cyclododecatriene (CDT) by selective hydrogenation as described, for instance, in EP 1 457 476 A1.

In the first step (step (i)) of the present method for epoxidizing a monoethylenically unsaturated organic compound, a first stream comprising the monoethylenically unsaturated organic compound is mixed with up to 25 wt.-% of a second stream comprising a primary alcohol, a secondary alcohol, a polyethylenically unsaturated organic compound or a combination of any of them, wherein wt.-% is based on the total weight of the combined first and second stream. The first stream may be mixed with from 1.0 wt.-% to 25 wt.-% of the second stream, preferably with from 1.0 wt.-% to less than 20 wt.-%, wherein wt.-% is based on the total weight of the combined first and second stream.

As used herein, the term "stream" refers to a flow of material, which can be a liquid, gas, or solid, moving through a process, preferably a continuous flow.

As used herein, the term "primary alcohol" relates to an organic compound in which a hydroxy group (-OH) is attached to a saturated primary carbon atom (R-CH₂-OH). Preference is given to a primary alcohol of an organic compound having a total of six to twenty carbon atoms, preferably a primary alcohol of an C11 or C12 organic compound, more preferably cycloundecanemethanol, 1-dodecanol, 1-undecanol or a combination thereof.

As used herein, the term "secondary alcohol" relates to an organic compound in which a hydroxy group (-OH) is attached to a saturated secondary carbon atom ((R¹)(R²)-CH-OH, wherein R¹ and R² may be the same or different carbon-containing group or together may form a cyclic structure). Preference is given to a secondary alcohol of an organic compound having a total of six to twenty carbon atoms, preferably a secondary alcohol of a cyclic organic compound having a total of six to twenty carbon atoms, more preferably a secondary alcohol of a cyclic C12 organic compound, most preferably cyclododecanol (CDOL).

As used herein, the term "polyethylenically unsaturated organic compound" refers to an organic compound having at least two carbon-carbon double bonds and/or at least two carbon-carbon triple bonds, preferably at least two carbon-carbon double bonds, more preferably exactly two or three carbon-carbon double bonds. Preference is given to an organic compound having a total of six to twenty carbon atoms, preferably a cyclic organic compound having a total of six to twenty carbon atoms, more preferably a cyclic polyethylenically unsaturated C12 compound, most preferably cyclododecadiene (CDD), cyclododecatriene (CDT), or a mixture thereof.

In the second step (step (ii)) of the present method for epoxidizing a monoethylenically unsaturated organic compound, the mixture obtained in step (i) is oxidized by at least one peroxide in the presence of a catalyst.

According to the present invention, the term "oxidation" is to be understood in the way it is generally understood by a skilled person in the field of organic synthesis and encompasses an epoxidation. In the oxidation according to step (ii) of the method of the present invention a peroxide is used as an oxidant. Suitable peroxides are known to those skilled in the art and include 3-chloroperoxybenzoic acid, peroxybenzoic acid, peroxyacetic acid, peroxybenzimidic acid, tert-butylhydroperoxide, dimethyldioxirane, potassium hydrogen peroxomonosulfate and hydrogen peroxide, wherein hydrogen peroxide is the preferred peroxide.

The oxidation in step (ii) of the method for epoxidizing a monoethylenically unsaturated organic compound is carried out in the presence of a catalyst. Suitable catalysts for the oxidation in step (ii) are known to those skilled in the art and can comprise homogeneous or heterogeneous catalyst systems. The catalyst used in step (ii) preferably is a homogeneous catalyst system. Preference is given to a homogeneous catalyst system comprising at least one derivative of a transition metal of Group IVb, Vb and Vlb in its highest oxidation state.

As used herein, the term "homogeneous catalyst system" is to be understood in the way it is generally understood by a skilled person in the field of organic synthesis, namely as a catalyst system used in homogeneous catalysis, wherein the catalyst is in the same phase as the reactants such as in case of a soluble catalyst in a solution. The homogeneous catalyst system catalyzes the oxidation of the organic compounds in the mixture obtained in step (i), such as the oxidation of the monoethylenically unsaturated organic compound, the primary alcohol, the secondary alcohol and the polyethylenically unsaturated organic compound.

As used herein, the term "transition metal of Group IVb" refers to an element that is in Group IVb of the CAS version of the Periodic Table of the Elements as is shown, for example, in the Handbook of Chemistry and Physics, 63^{rd} edition (1983), corresponding to Group 4 in the actual IUPAC numbering. Likewise, the terms "transition metal of Group Vb" and "transition metal of Group Vlb" refer to an element that is in Group Vb and Vlb, respectively, of the CAS version of the Periodic Table of the Elements as is shown, for example, in the Handbook of Chemistry and Physics, 63^{rd} edition (1983), corresponding to Group 5 and 6, respectively, in the actual IUPAC numbering.

The at least one derivative of the transition metal of Group IVb, Vb and Vlb preferably may be selected from a derivative of tungsten (oxidation state 6), molybdenum (oxidation state 6) and vanadium (oxidation state 5). Suitable derivatives include, for example, oxides, mixed oxides, oxygen-containing acids, salts of oxygen-containing acids, carbonyl derivatives, sulfides, chlorides, oxychlorides and alkanoates of tungsten, molybdenum and/or vanadium.

Preference is given to derivatives selected from salts of tungstic acid (H₂WO₄) or molybdic acid (H₂MoO₄), or homo- or heteropolyoxometalates formed therefrom. Special preference is given to derivatives selected from alkali or alkaline earth metal salts of H₂WO₄ or H₂MoO₄, or homo- or heteropolyoxometalates formed therefrom, in particular to Na₂WO₄ or a homo- or heteropolyoxometalate formed therefrom.

A polyoxometalate is a polyatomic ion, usually an anion, comprising three or more transition metal oxyanions linked together by shared oxygen atoms to form closed 3-dimensional frameworks. Homopolyoxometalates are composed of only one kind of metal and oxygen, while heteropolyoxometalates are composed of one or more metals, oxygen and eventually a main group oxyanion, such as a phosphate or a silicate.

These derivatives can be formed or converted into the catalytically active species *in situ.* For instance, the catalyst system can further comprise phosphoric acid, a salt of phosphoric acid or a combination of both. For example, sodium tungstate can be used in combination with phosphoric acid to form a catalytically active polyoxometalate in situ. Phosphoric acid or salts thereof can, for instance, be used as a stabilizer for hydrogen peroxide and thus, if hydrogen peroxide is used as the oxidant, be added to the reaction mixture in this form in combination with the hydrogen peroxide.

The method for epoxidizing a monoethylenically unsaturated organic compound according to the present invention may further comprise a third step (step (iii)) of separating compounds having a boiling point (bp) higher than the boiling point of the epoxidized monoethylenically unsaturated organic compound (so-called high boilers). The separation may be performed by distillation, preferably by flash-distillation. As used herein, the term "flash-distillation" refers to a separation process in which a liquid mixture is rapidly vaporized (or "flashed") into vapor and liquid phases at a specific pressure and temperature in order to separate components based on their boiling points.

If the separation is carried out by means of distillation, the compounds having a boiling point higher than the boiling point of the epoxidized monoethylenically unsaturated organic compound remain in the distillation residue, while the distillate obtained by the distillation comprises the epoxidized monoethylenically unsaturated organic compound. The distillate may further comprise a derivative of the monoethylenically unsaturated organic compound comprising a ketone functionality, also referred to herein as a corresponding ketone, wherein said derivative corresponds to the epoxide of the monoethylenically unsaturated organic compound except that it contains a ketone functional group (R¹-(C=O)-CR²R³) instead of the epoxide functional group as shown below

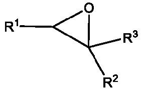

Wherein R¹ is a carbon-containing group and R² and R³ each independently may be hydrogen or a carbon-containing group and the carbon-containing groups of R¹, R² and r3 may be the same or different or together may form a cyclic structure.

When using flash-distillation, the distillate may comprise 95 wt.-% of the mixture obtained after oxidation in step (ii), wherein wt.-% is based on the total weight of the mixture after oxidation.

For instance, if CDEN is epoxidized in the presence of CDOL, CDD, CDT, cycloundecanemethanol, 1-dodecanol and/or 1-undecanol, the epoxidized product CDAN-epoxide (bp = 274 - 276 °C) can be separated together with the oxidation product CDON (bp = 277 °C) from the high boiling products resulting from the oxidation of CDD, CDT, cycloundecanemethanol, 1-dodecanol and/or 1-undecanol, i.e., CDAN-diepoxide, CDAN-triepoxide, cycloundecanecarboxylic acid, dodecanoic acid (bp = 299 °C) and undecanoic acid (bp = 284 °C). The boiling points used herein refer to the boiling points of the compounds at the normal pressure of 1 atm (1013 hPa).

The distillate obtained in step (iii) of the method described herein may comprise the epoxidized monoethylenically unsaturated organic compound in an amount of at least 50 wt.-%, preferably in an amount of at least 75 wt.-% and further comprises a derivative of the monoethylenically unsaturated organic compound comprising a ketone functionality in an amount of at least 1.0 wt.-%, preferably of at least 2.0 wt.-%, wherein wt.-% is based on the total weight of the distillate.

The distillate obtained in step (iii) comprising the epoxidized monoethylenically unsaturated organic compound may be used as a reactant in a subsequent reaction without further purification. As used herein, the phrase "without further purification" means that the distillate is fed directly into a subsequent reaction without an intermediate step. The subsequent reaction may be a reaction sequence comprising more than one reaction step. The subsequent reaction may preferably comprise a ring-opening of the epoxide functional group (oxirane) of the epoxidized monoethylenically unsaturated organic compound. Special preference is given to a subsequent reaction comprising the rearrangement of the epoxidized monoethylenically unsaturated organic compound to a corresponding ketone including the ring-opening of the epoxide functional group. The subsequent reaction may further comprise an oximation of the ketone obtained in the rearrangement to a corresponding oxime and optionally a rearrangement of the obtained oxime to a corresponding amine.

For instance, the distillate obtained in step (iii) comprising the epoxidized monoethylenically unsaturated organic compound can be used without further purification in a rearrangement reaction of the epoxidized monoethylenically unsaturated organic compound to a corresponding ketone including the step of ring-opening of the epoxide functional group. The rearrangement may be performed in the presence of catalyst system comprising a noble metal, for instance, ruthenium, palladium or platinum, and the metal oxide(s) titanium dioxide and/or zirconium dioxide as described, for instance, in EP 2 772 478 A1 or EP 2 980 071 A1. The obtained ketone may then be subjected to an oximation to form the corresponding oxime. This oximation reaction may be carried out using ammonia and hydrogen peroxide in the presence of a titanium silicalite catalyst as described, for example, in US 2003/0100795 A1. The obtained oxime may afterwards be subjected to a rearrangement reaction to convert the oxime to the corresponding amide. The rearrangement of the oxime to the corresponding amide may be performed by the Beckmann rearrangement as described for example in EP 2 013 162 B1.

The second stream used in step (i) of the method according to the present invention may be a waste stream, preferably a waste stream from another reaction (step), such as one of steps (ii) or (iv) of the method for synthesizing a lactam described in more detail below. The term "waste stream" as used herein, refers to a stream obtained from a process step, wherein the stream has a lower economic value per kg than another product stream obtained in the same process step. For instance, a waste stream may be a stream obtained from a process step carrying out at least one preceding reaction after separating at least a part of the product of the reaction from the stream. A waste stream of a reaction, as used herein, may contain products, by-products and or starting material of the performed reaction.

The second stream may be a waste stream from a rearrangement reaction of an epoxidized monoethylenically unsaturated organic compound to the corresponding ketone, a waste stream from a rearrangement reaction of an oxime to the corresponding amide, a waste stream from an oxidation-dehydrogenation process of a saturated organic compound to a ketone via a secondary alcohol or any combination of the foregoing. The oxidation-dehydrogenation reaction may be used to oxidize CDAN under Bashkirov conditions to CDOL, followed by dehydrogenation of CDOL to CDON using, for instance, copper(II)-oxide as described e.g., in EP 2 980 071 A1.

The second stream used in step (i) of the method described herein may comprise the primary alcohol, if present, in an amount of from 0.01 to 20 wt.-%, preferably of from 0.01 to 10 wt.-%, wherein wt.-% is based on the total weight of the second stream. Moreover, the second stream may comprise the secondary alcohol, if present, in an amount of from 20 to 99 wt.-%, preferably of from 50 to 99 wt.-%, wherein wt.-% is based on the total weight of the second stream. The second stream may comprise the polyethylenically unsaturated organic compound, if present, in an amount of from 0.01 to 20 wt.-%, preferably of from 0.01 to less than 5 wt.-%, wherein wt.-% is based on the total weight of the second stream.

The second stream may further comprise a derivative of the monoethylenically unsaturated organic compound comprising a ketone functionality, wherein said derivative corresponds to the epoxide of the monoethylenically unsaturated organic compound except that it contains a ketone functional group (R¹-(C=O)-CR²R³) instead of the epoxide functional group as shown below 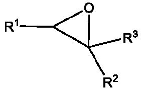
wherein R¹, R² and R³ are and defined above,
and/or a derivative of an organic compound having a total of six to twenty carbon atoms comprising an ether functionality. The second stream may comprise the derivative of the monoethylenically unsaturated organic compound comprising a ketone functionality in an amount of from 50 to 99 wt.-%, preferably of from 70 to 95 wt.-%, and/or the derivative of an organic compound having a total of six to twenty carbon atoms comprising an ether functionality in an amount of from 0.01 to 5 wt.-%, preferably of from 0.01 to less than 1.0 wt.-%, wherein wt.-% is based on the total weight of the second stream.

The second stream used in step (i) may also be a combination of at least two of the waste streams described above. In this case the previously described amounts for each compound refer to their amount in the individual waste streams. The actual amounts of the primary alcohol, the secondary alcohol, the polyethylenically unsaturated organic compound and/or the derivative of the monoethylenically unsaturated organic compound comprising a ketone functionality in the resulting combined stream vary dependent on the mixing ratio of the different waste streams forming the second stream. In any case, no more than 25 wt.-% of said combined waste streams are mixed as a second stream with the first stream in step (i) of the present method for epoxidizing a monoethylenically unsaturated organic compound.

The method for epoxidizing a monoethylenically unsaturated organic compound as described herein may further comprise a step of separating the catalyst from the reaction mixture of step (ii) before conducting step (iii). The catalyst may be separated using membrane filtration, distillation or a combination of both. After the separation of the catalyst, the catalyst may be reactivated for re-use. Suitable methods for separating or reactivating a catalyst system used in an oxidation reaction are described for instance in EP 2 946 831 A2 and WO 2018/002114 A1.

The method for epoxidizing a monoethylenically unsaturated organic compound according to the present invention may be carried out as a batch process or as a continuous process, wherein a continuous process is preferred. Special preference is given to the method carried out as a continuous process in a cascade of stirred tank reactors. The first and second stream are fed to a first stirred tank reactor as separate streams which only are mixed in said tank reactor and/or as a preformed mixture. The method may be carried out in the apparatus described herein below.

For instance, the method for epoxidizing a monoethylenically unsaturated organic compound described herein can be used for the epoxidation of CDEN to CDAN-epoxide and thus in the process for synthesizing laurolactam. In this case, a first stream comprising CDEN is mixed in step (i) of the present method with up to 25 wt.-% of a second stream comprising at least one of cycloundecanemethanol, 1-dodecanol, 1-undecanol, CDOL, CDT and CDD and the mixture is oxidized in step (ii) by at least one peroxide in the presence of a catalyst. While CDEN is converted into CDAN-epoxide in the oxidation, CDOL can be converted into CDON under the same reaction conditions employed for said epoxidation reaction. Moreover, the primary alcohols can be converted into the corresponding carboxylic acids and CDT and CDD can be converted into the corresponding polyepoxide compounds. The obtained carboxylic acids and polyepoxides represent high boilers. Thus, in the separation of step (iii) a distillate comprising CDAN-epoxide and CDON but being essentially free from primary alcohols, CDOL, CDT, CDD and their aforementioned oxidation products can be obtained. As used herein, the term "essentially free" means that less than 0.1 wt.-% of each of the respective compounds is comprised in the distillate. This distillate can be used without further purification in the rearrangement reaction of CDAN-epoxide to CDON and the CDON can afterwards be used in the process for synthesizing laurolactam. In this case, the second stream can be the waste stream of at least one reaction of the reaction cascade for synthesizing laurolactam, such as the waste stream from the rearrangement of CDAN-epoxide to CDON, the waste stream from the Beckmann rearrangement or from a process wherein CDAN is oxidized to CDOL followed by dehydrogenation.

The present invention also relates to an apparatus for carrying out the method for epoxidizing a monoethylenically unsaturated organic compound as described herein. The apparatus comprises a first stirred tank reactor or a first series of stirred tank reactors coupled to each other, being adapted to carry out the oxidation of step (ii); a second stirred tank reactor or a second series of stirred tank reactors coupled to each other, being adapted to carry out a further reaction of the oxidation product of step (ii) or a subsequent reaction product thereof, thereby producing a waste stream comprising a primary alcohol, a secondary alcohol, a polyethylenically unsaturated organic compound or a combination of any of them; and (iii) a pipeline connecting the second stirred tank reactor or series of stirred tank reactors with the first stirred tank reactor or series of stirred tank reactors to direct the waste stream produced in the second stirred tank reactor or series of stirred tank reactors to the first stirred tank reactor or series of stirred tank reactors. These series of stirred tank reactors preferably comprise at least two stirred tank reactors, more preferably three to five stirred tank reactors.

The present invention further relates to a method for synthesizing a lactam, comprising the steps of (i) epoxidizing a cyclic monoethylenically unsaturated organic compound to an epoxide; (ii) rearranging the epoxide to a ketone; (iii) converting the ketone to an oxime; and (iv) rearranging the oxime to the lactam; wherein step (i) comprises the epoxidation according to the method described herein.

The method for synthesizing a lactam is particularly suitable for synthesizing laurolactam. The cyclic monoethylenically unsaturated organic compound preferably may comprise 6 to 20 carbon atoms, more preferably 12 carbon atoms and most preferably is CDEN.

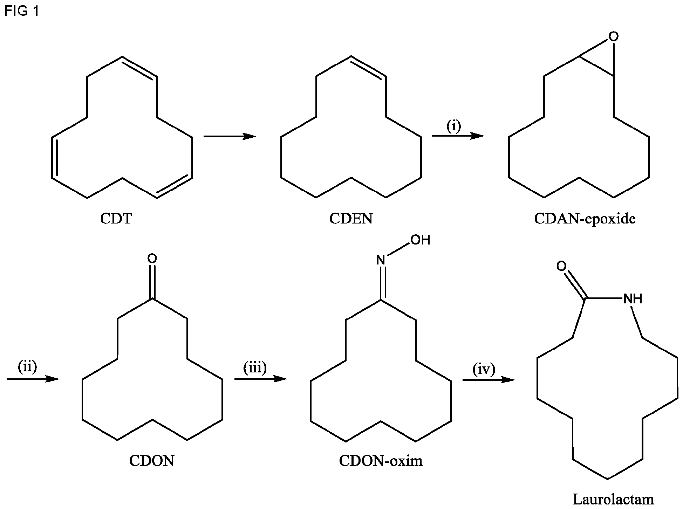

The method for synthesizing a lactam is schematically illustrated for the cyclic monoethylenically unsaturated organic compound CDEN in Figure 1. Prior to the method for synthesizing a lactam, the starting material CDEN may, for instance, be prepared by selective hydrogenation of CDT. In the first step (step i) of the method for synthesizing a lactam, the organic compound is oxidized to the corresponding epoxide. This epoxidation is carried out according to the method described herein above. The epoxidized compound is subsequently rearranged, for instance, in the presence of a catalyst system comprising a noble metal and a metal oxide, to the corresponding ketone in step (ii). During said rearrangement or subsequent thereto, hydrogen can be added, so that the corresponding alcohol is formed. If the ketone is present in a mixture with the alcohol derivative, a dehydrogenation of the alcohol to the ketone can take place. In step (iii), the ketone is then converted to an oxime and finally the lactam is obtained by subsequent Beckmann rearrangement in step (iv) using e.g., sulfuric acid or cyanuric chloride. The rearrangement of the epoxide to the ketone and the subsequent steps are disclosed, for instance, in EP 2 772 478 A1 and EP 2 980 071 A1. The resulting lactam can be subjected to further processing by polycondensation to give polyamides. A method for synthesizing laurolactam from CDEN via 1,2-epoxycyclododecane (CDAN epoxide) as an intermediate is particularly preferred. The obtained laurolactam can be polymerized to nylon 12 afterwards. The starting material CDEN can be obtained from CDT by selective hydrogenation.

The present invention further relates to a monophasic or biphasic composition comprising an organic phase, wherein the organic phase comprises an epoxidized monoethylenically unsaturated organic compound in an amount of from 50 to 99 wt.-%, preferably of from 75 to 98 wt.-%, a derivative of the monoethylenically unsaturated organic compound comprising a ketone functionality, wherein said derivative corresponds to the epoxide of the monoethylenically unsaturated organic compound except that it contains a ketone functional group (R¹-(C=O)-CR²R³) instead of the epoxide functional group as shown below 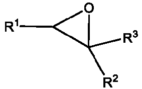 wherein R¹, R² and R³ are as defined above, in an amount of from 1.0 to 20 wt.-%, preferably of from 2.0 to 20 wt.-%, and tungsten in an amount of from 500 to 5000 ppm, preferably of from 1000 to 3000 ppm; wherein wt.-% and ppm are based on the total weight of the organic phase.

The term "biphasic composition" as used herein refers to a mixture comprising two liquid phases being immiscible at ambient temperature, namely an aqueous and an organic phase. Unless stated otherwise, "ambient temperature" or "room temperature" as used herein refers to a temperature of 23 °C.

The epoxidized monoethylenically unsaturated organic in the organic phase of the monophasic or biphasic composition described herein may comprise epoxycyclododecane (CDAN-epoxide) and the derivative of the monoethylenically unsaturated organic compound comprising a ketone functionality may comprise cyclododecanone (CDON).

The amount of the epoxidized monoethylenically unsaturated organic compound, such as CDAN-epoxide and of the derivative of the monoethylenically unsaturated organic compound comprising a ketone functionality, such as CDON can, for example, be determined by gas chromatography with a flame ionization detector (GC-FID) using, for instance, a GC-2014 gas chromatograph from Shimadzu Deutschland GmbH (Duisburg, Germany). The determination can be carried out as follows: a sample of the reaction mixture of the oxidation reaction is centrifuged at room temperature (23 °C) for 1 min at 4000 rpm for obtaining a complete phase separation. 100 mg of the organic phase is weighed into a GC vial and diluted with standard solution by a factor of 10. The standard solution comprises 1 wt.-% of tetradecane in acetone. The measurement is performed using a Shimadzu GC-2014 gas chromatograph with a Supelcowax-10 column (length of 60 m, diameter of 0.32 mm and film thickness of 0.25 µm) with a flame ionization detector and a SPL-10 Split Injector. The sample is injected at 150 °C with a split of 1:10. The initial column temperature is 180 °C, which is held for 10 min, then increased with a temperature ramp of 5 K/min to 200 °C and kept at this temperature for 35 min. The amount of the products is determined (quantified) by comparing the area of the products with the area of tetradecane as internal standard in correlation with the initial weight and a response factor, which is determined beforehand by recovery of the pure substances in a concentration series.

The amount of the transition metal, such as tungsten, in the monophasic or biphasic composition can be determined by X-ray fluorescence spectroscopy using, for instance, a SPECTRO XEPOS spectrometer of type 16004851 from SPECTRO Analytical Instruments GmbH (Kleve, Germany). For a biphasic composition, a sample is centrifuged at room temperature (23 °C) for 1 min at 4000 rpm for complete phase separation and the phases are separated by pipetting. For a monophasic composition this step is omitted. 5 g of the organic phase is transferred into a single usage cuvette having a diameter of 32 mm from SPECTRO Analytical Instruments GmbH (Kleve, Germany). The concentration is determined via X-ray fluorescence analysis using a SPECTRO XEPOS spectrometer, type 16004851 from SPECTRO Analytical Instruments GmbH (Kleve, Germany). The sample chamber is inertized with helium. The amount of the transition metal is determined using a method calibrated with the pure substances, for instance sodium tungstate and phosphoric acid (75%).

Using these absolute methods as references the concentration of epoxide and ethylenically unsaturated organic compound can also be quantified online during the reaction using a Kaiser Optical Systems Rxn2 Raman analyzer from Endress+Hauser Group Services AG (Reinach, Switzerland) with a 785 nm laser, and immersion short focus probes and indirect hard modeling to quantify CDEN and CDAN-epoxide concentration and a partial least-square model for tungstate and hydrogen peroxide employing the Peaxact Software from S-PACT (Aachen, Germany). An online measurement as used herein refers to a method taking place continuously but not, as for inline measurements, directly in the process but, for example, in a bypass, through which the reactor content is continuously passed.

The following clauses summarize some aspects of the present invention:
In a first aspect the present invention relates to a method for epoxidizing a monoethylenically unsaturated organic compound, comprising the steps of (i) mixing a first stream comprising the monoethylenically unsaturated organic compound with up to 25 wt.-% of a second stream comprising a primary alcohol, a secondary alcohol, a polyethylenically unsaturated organic compound or a combination of any of them, wherein wt.-% is based on the total weight of the combined first and second stream; (ii) oxidizing the mixture obtained in step (i) by at least one peroxide in the presence of a catalyst.

In a second aspect the present invention relates to the method of the first aspect, further comprising a step (iii) of separating compounds having a boiling point higher than the boiling point of the epoxidized monoethylenically unsaturated organic compound.

In a third aspect the present invention relates to the method of the second aspect, wherein the compounds are separated in step (iii) by distillation, preferably by flash-distillation.

In a fourth aspect the present invention relates to the method of the third aspect, wherein the distillate obtained by distillation in step (iii) comprises the epoxidized monoethylenically unsaturated organic compound.

In a fifth aspect the present invention relates to the method of the fourth aspect, wherein the distillate is used as a reactant in a subsequent reaction without further purification.

In a sixth aspect the present invention relates to the method of the fifth aspect, wherein the subsequent reaction comprises a ring-opening of the epoxide functional group of the epoxidized monoethylenically unsaturated organic compound.

In a seventh aspect the present invention relates to the method of the sixth aspect, wherein the subsequent reaction comprises the rearrangement of the epoxidized monoethylenically unsaturated organic compound to a corresponding ketone.

In an eighth aspect the present invention relates to the method of any of aspects six or seven, wherein the subsequent reaction further comprises an oximation of the ketone to a corresponding oxime.

In a ninth aspect the present invention relates to the method of the eighth aspect, wherein the subsequent reaction further comprises a rearrangement of the oxime to an amide.

In a tenth aspect the present invention relates to the method of any of the fourth to ninth aspect, wherein the distillate obtained in step (iii) comprises the epoxidized monoethylenically unsaturated organic compound in an amount of at least 50 wt.-% and further comprises a derivative of the monoethylenically unsaturated organic compound comprising a ketone functionality in an amount of at least 1.0 wt.-%, wherein wt.-% is based on the total weight of the distillate.

In an eleventh aspect the present invention relates to the method of the tenth aspect, wherein the distillate comprises the epoxidized monoethylenically unsaturated organic compound in an amount of at least 50 wt.-% and the derivative of the monoethylenically unsaturated organic compound comprising a ketone functionality in an amount of at least 2.0 wt.-%.

In a twelfth aspect the present invention relates to the method of the tenth aspect, wherein the distillate comprises the epoxidized monoethylenically unsaturated organic compound in an amount of at least 75 wt.-% and the derivative of the monoethylenically unsaturated organic compound comprising a ketone functionality in an amount of at least 1.0 wt.-%.

In a thirteenth aspect the present invention relates to the method of the tenth aspect, wherein the distillate comprises the epoxidized monoethylenically unsaturated organic compound in an amount of at least 75 wt.-% and the derivative of the monoethylenically unsaturated organic compound comprising a ketone functionality in an amount of at least 2.0 wt.-%.

In a fourteenth aspect the present invention relates to the method of any of the preceding aspects, wherein the second stream is a waste stream, preferably a waste stream from another reaction.

In a fifteenth aspect the present invention relates to the method of the fourteenth aspect, wherein the second stream is a waste stream from a rearrangement reaction of an epoxidized monoethylenically unsaturated organic compound to the corresponding ketone, a waste stream from a rearrangement reaction of an oxime to the corresponding amide, a waste stream from an oxidation-dehydrogenation process of a saturated organic compound to a ketone via a secondary alcohol or any combination of the foregoing.

In a sixteenth aspect the present invention relates to the method of any of the preceding aspects, wherein the monoethylenically unsaturated organic compound is an organic compound having a total of six to twenty carbon atoms, preferably a cyclic organic compound having a total of six to twenty carbon atoms, more preferably a cyclic monoethylenically unsaturated C12 compound, most preferably cyclododecene (CDEN).

In a seventeenth aspect the present invention relates to the method of any of the preceding aspects, wherein the primary alcohol is a primary alcohol of an organic compound having a total of six to twenty carbon atoms, preferably a primary alcohol of an C11 or C12 organic compound, more preferably cycloundecanemethanol, 1-dodecanol, 1-undecanol or a combination thereof.

In an eighteenth aspect the present invention relates to the method of any of the preceding aspects, wherein the secondary alcohol is a secondary alcohol of an organic compound having a total of six to twenty carbon atoms, preferably a secondary alcohol of a cyclic organic compound having a total of six to twenty carbon atoms, more preferably a secondary alcohol of a cyclic C12 organic compound, most preferably cyclododecanol (CDOL).

In a nineteenth aspect the present invention relates to the method of any of the preceding aspects, wherein the polyethylenically unsaturated organic compound is an organic compound having a total of six to twenty carbon atoms, preferably a cyclic organic compound having a total of six to twenty carbon atoms, more preferably a cyclic polyethylenically unsaturated C12 compound, most preferably cyclododecadiene (CDD), cyclododecatriene (CDT), or a mixture thereof.

In a twentieth aspect the present invention relates to the method of any of the preceding aspects, wherein the second stream comprises the primary alcohol in an amount of from 0.01 to 20 wt.-%, preferably of from 0.01 to 10 wt.-%, wherein wt.-% is based on the total weight of the second stream.

In a twenty-first aspect the present invention relates to the method of any of the preceding aspects, wherein the second stream comprises the secondary alcohol in an amount of from 20 to 99 wt.-%, preferably of from 50 to 99 wt.-%, wherein wt.-% is based on the total weight of the second stream.

In a twenty-second aspect the present invention relates to the method of any of the preceding aspects, wherein the second stream comprises the polyethylenically unsaturated organic compound in an amount of from 0.01 to 20 wt.-%, preferably of from 0.01 to less than 5 wt.-%, wherein wt.-% is based on the total weight of the second stream.

In a twenty-third aspect the present invention relates to the method of any of the preceding aspects, wherein the second stream further comprises a derivative of the monoethylenically unsaturated organic compound comprising a ketone functionality in an amount of from 50 to 99 wt.-%, preferably of from 70 to 95 wt.-%, wherein wt.-% is based on the total weight of the second stream.

In a twenty-fourth aspect the present invention relates to the method of any of the preceding aspects, wherein the second stream further comprises a derivative of an organic compound having a total of six to twenty carbon atoms comprising an ether functionality in an amount of from 0.01 to 5 wt.-%, preferably of from 0.01 to less than 1.0 wt.-%, wherein wt.-% is based on the total weight of the second stream.

In a twenty-fifth aspect the present invention relates to the method of any of the preceding aspects, wherein the first stream is mixed in step (i) with from 1.0 wt.-% to 25 wt.-% of the second stream, preferably from 1.0 wt.-% to less than 20 wt.-%, wherein wt.-% is based on the total weight of the combined first and second stream.

In a twenty-sixth aspect the present invention relates to the method of any of the preceding aspects, wherein the catalyst is a homogeneous catalyst system.

In a twenty-seventh aspect the present invention relates to the method of the twenty-sixth aspect, wherein the homogeneous catalyst system comprises at least one derivative of a transition metal of Group IVb, Vb and Vlb in its highest oxidation state.

In a twenty-eighth aspect the present invention relates to the method of the twenty-seventh aspect, wherein the at least one derivative of the transition metal of Group IVb, Vb and VIb is selected from a derivative of tungsten, molybdenum and vanadium, wherein the derivative preferably is selected from salts of H₂WO₄ or H₂MoO₄, or homo- or heteropolyoxometalates formed therefrom, more preferably from alkali or alkaline earth metal salts of H₂WO₄ or H₂MoO₄, or homo- or heteropolyoxometalates formed therefrom, and most preferably is Na₂WO₄ or a homo- or heteropolyoxometalate formed therefrom.

In a twenty-ninth aspect the present invention relates to the method of any of the twenty-seventh or twenty-eighth aspect, wherein the homogeneous catalyst system further comprises phosphoric acid and/or a salt thereof.

In a thirtieth aspect the present invention relates to the method of any of the preceding claims, wherein the method further comprises separating the catalyst from the reaction mixture of step (ii) before conducting step (iii).

In a thirty-first aspect the present invention relates to the method of the thirtieth aspect, wherein the catalyst is separated using membrane filtration and/or distillation.

In a thirty-second aspect the present invention relates to the method of any of the thirtieth or thirty-first aspect, wherein the catalyst system is reactivated for re-use.

In a thirty-third aspect the present invention relates to the method of any of the preceding aspects, wherein the method is carried out as a continuous process.

In a thirty-fourth aspect the present invention relates to a method for synthesizing a lactam comprising the steps of: (i) epoxidizing a cyclic monoethylenically unsaturated organic compound to an epoxide; (ii) rearranging the epoxide to a ketone; (iii) converting the ketone to an oxime; and (iv) rearranging the oxime to the lactam; wherein step (i) comprises the epoxidation according to the method of any of the first to thirty-third aspects.

In a thirty-fifth aspect the present invention relates to an apparatus for carrying out the method according to any of the first to thirty-third aspects, wherein the apparatus comprises (i) a first stirred tank reactor or a first series of stirred tank reactors coupled to each other, being adapted to carry out the oxidation of step (ii); (ii) a second stirred tank reactor or a second series of stirred tank reactors coupled to each other, being adapted to carry out a further reaction of the oxidation product of step (ii) or a subsequent reaction product thereof, thereby producing a waste stream comprising a primary alcohol, a secondary alcohol, a polyethylenically unsaturated organic compound or a combination of any of them; and (iii) a pipeline connecting the second stirred tank reactor or series of stirred tank reactors with the first stirred tank reactor or series of stirred tank reactors to direct the waste stream produced in the second stirred tank reactor or series of stirred tank reactors to the first stirred tank reactor or series of stirred tank reactors.

In a thirty-sixth aspect the present invention relates to a monophasic or biphasic composition comprising an organic phase, wherein the organic phase comprises an epoxidized monoethylenically unsaturated organic compound in an amount of from 50 to 99 wt.-%, preferably of from 75 to 98 wt.-%, a derivative of the monoethylenically unsaturated organic compound comprising a ketone functionality in an amount of from 1.0 to 20 wt.-%, preferably of from 2.0 to 20 wt.-%, and tungsten in an amount of from 500 to 5000 ppm, preferably of from 1000 to 3000 ppm; wherein wt.-% and ppm are based on the total weight of the organic phase.

In a thirty-seventh aspect the present invention relates to the composition of the thirty-sixth aspect, wherein the organic phase comprises the epoxidized monoethylenically unsaturated organic compound in an amount of from 50 to 99 wt.-%, the derivative of the monoethylenically unsaturated organic compound comprising a ketone functionality in an amount of from 1.0 to 20 wt.-%, and tungsten in an amount of from 500 to 5000 ppm.

In a thirty-eighth aspect the present invention relates to the composition of the thirty-sixth aspect, wherein the organic phase comprises the epoxidized monoethylenically unsaturated organic compound in an amount of from 50 to 99 wt.-%, the derivative of the monoethylenically unsaturated organic compound comprising a ketone functionality in an amount of from 1.0 to 20 wt.-%, and tungsten in an amount of from 1000 to 3000 ppm.

In a thirty-ninth aspect the present invention relates to the composition of the thirty-sixth aspect, wherein the organic phase comprises the epoxidized monoethylenically unsaturated organic compound in an amount of from 50 to 99 wt.-%, the derivative of the monoethylenically unsaturated organic compound comprising a ketone functionality in an amount of from 2.0 to 20 wt.-%, and tungsten in an amount of from 500 to 5000 ppm.

In a fortieths aspect the present invention relates to the composition of the thirty-sixth aspect, wherein the organic phase comprises the epoxidized monoethylenically unsaturated organic compound in an amount of from 50 to 99 wt.-%, the derivative of the monoethylenically unsaturated organic compound comprising a ketone functionality in an amount of from 2.0 to 20 wt.-%, and tungsten in an amount of from 1000 to 3000 ppm.

In a forty-first aspect the present invention relates to the composition of the thirty-sixth aspect, wherein the organic phase comprises the epoxidized monoethylenically unsaturated organic compound in an amount of from 75 to 98 wt.-%, the derivative of the monoethylenically unsaturated organic compound comprising a ketone functionality in an amount of from 1.0 to 20 wt.-%, and tungsten in an amount of from 500 to 5000 ppm.

In a forty-second aspect the present invention relates to the composition of the thirty-sixth aspect, wherein the organic phase comprises the epoxidized monoethylenically unsaturated organic compound in an amount of from 75 to 98 wt.-%, the derivative of the monoethylenically unsaturated organic compound comprising a ketone functionality in an amount of from 1.0 to 20 wt.-%, and tungsten in an amount of from 1000 to 3000 ppm.

In a forty-third aspect the present invention relates to the composition of the thirty-sixth aspect, wherein the organic phase comprises the epoxidized monoethylenically unsaturated organic compound in an amount of from 75 to 98 wt.-%, the derivative of the monoethylenically unsaturated organic compound comprising a ketone functionality in an amount of from 2.0 to 20 wt.-%, and tungsten in an amount of from 500 to 5000 ppm.

In a forty-fourth aspect the present invention relates to the composition of the thirty-sixth aspect, wherein the organic phase comprises the epoxidized monoethylenically unsaturated organic compound in an amount of from 75 to 98 wt.-%, the derivative of the monoethylenically unsaturated organic compound comprising a ketone functionality in an amount of from 2.0 to 20 wt.-%, and tungsten in an amount of from 1000 to 3000 ppm.

In a forty-fifth aspect the present invention relates to the composition of any of the thirty-sixth to forty-fourth aspect, wherein the epoxidized monoethylenically unsaturated organic compound comprises cyclododecane-epoxide and the derivative of the monoethylenically unsaturated organic compound comprising a ketone functionality comprises cyclododecanone.

It is assumed that a person skilled in the art can make very extensive use of the above description. The preferred embodiments are therefore to be interpreted merely as descriptive disclosure, and certainly not as disclosure that is in any way limiting. The present invention is elucidated in detail hereinafter with reference to examples which are provided herein for purpose of illustration only, and are not intended to be limiting unless otherwise specified. Alternative embodiments of the present invention are obtainable analogously.

### Figures

FIG. 1 shows a reaction scheme for the reaction sequence underlying the method for synthesizing a lactam for the cyclic monoethylenically unsaturated organic compound CDEN.

### Examples

### Reference Example

An epoxidation of cyclic unsaturated C12 compounds was carried out in a continuous process in a cascade of three stirred tank reactors. The cascade comprised two reactors each having a 5 liter nominal capacity and, as a final stage, a third stirred tank reactor having a 25 liter nominal capacity. The content of the first two reactors was heated in an oil bath to 90 °C and that of the final reactor was heated in an oil bath to 80 °C.

To the first reactor 1.5 kg/h of cyclic unsaturated C12 compound (94 wt.-% CDEN and 6 wt.-% of CDAN), Adogen^{®} 464 methyl sulfate (PTC), sodium tungstate, phosphoric acid, sulfuric acid and a 60% H₂O₂ solution was fed. The pH value of the reaction mixture was adjusted to a pH of 1.60 by adding sulfuric acid.

The reaction mixture was passed into a second reactor. In addition, a further quantity of H₂O₂ was metered into the second reactor. In total, a ratio of 1.01 to 1.06 mol H₂O₂ per mol of CDEN was added to the first and second reactor.

The biphasic reaction mixture was passed from the second reactor into the third reactor and from there into a phase separation vessel to let the organic phase separate from the aqueous phase. The organic phase was supplied to a continuous membrane system using a pump and the aqueous phase was discarded. The residence time in each of the first and second reactor was approximately 2.5 h, while in the third reactor it was approximately 12.5 h. Residence time, as used herein, is the total average amount of time a discrete quantity of reagent spends inside the reactor. For an ideal continuously stirred-tank reactor, the theoretical residence time is equal to the reactor volume divided by the fluid flow rate.

The organic phase was fed to a membrane unit employing a oNF-2^{®} from Borsig Membrane Technology GmbH (Gladbeck, Germany) operating at 60 °C and a transmembrane-pressure of 40 bar. The organic phase was separated into permeate and retentate in a way such that 10 wt% of the feed was obtained as retentate and 90 wt% as permeate. The membrane was used as 2,5"x20" spiral-wound element to provide a sufficient permeate flow to process the feedflow of the organic phase and to provide an excess permeate-flow at the same time. The excess permeate-flow not needed for the permeate access was recycled to the feed.

The retentate was fed into a further stirred tank with a nominal capacity of 5 liter.

A 1.0 M aqueous sodium hydroxide solution was added and the biphasic mixture was adjusted to a pH of 8.5. The pH value was monitored using an online pH electrode and controlled with manual measurements using a Knick MEMO SES SE55X/1-NMSN sensor and a Knick Portavo 940X Multi 84461/205892 pH meter both from Knick Elektronische Messgeräte GmbH & Co. KG (Berlin, Germany).

The biphasic mixture having a pH of 8.5 was transferred into a stirred vessel with a nominal capacity of 5 liter referred to as hydrolysis reactor. Subsequently, the reaction mixture was recycled into the first reactor of the cascade.

The concentrations of CDEN, CDAN-epoxide and tungstate were monitored online employing Raman as described above and offline samples were taken every 24 hours until the reaction reaches a steady state and concentrations did not change anymore.

The conversion of CDEN to CDAN-epoxide was determined after reaching the steady state using the amounts determined by gas chromatography with a flame ionization detector (GC-FID) as described above.

In the Reference Example, a conversion rate of CDEN of 97% was achieved.

### Example 1

In Example 1 an epoxidation of cyclic unsaturated C12 compounds was carried out using the same conditions as described in the Reference Example above, except that in addition to the cyclic unsaturated C12 compound (94 wt.-% CDEN and 6 wt.-% of CDAN) 10 wt.-% of a second stream comprising 45 wt.-% CDOL, 34 wt.-% CDON and 5 wt.-% of a mixture of cycloundecanemethanol and 1-dodecanol was fed to the first reactor.

The conversion of CDEN was determined as described above using GC-FID. In addition, the conversion of CDOL to CDON was determined using GC-FID as described above. In Example 1, a conversion rate of CDEN to CDAN-epoxide of 97% and a conversion rate of CDOL to CDON of 75% were achieved.

### Example 2

In Example 2 an epoxidation of cyclic unsaturated C12 compounds was carried out using the same conditions as described in Example 1, except that the residence time in all three reactors was doubled by reducing the feed rates by half.

The conversion rates of CDEN to CDAN-epoxide and CDOL to CDON were determined as described above. In Example 2, a conversion rate of CDEN to CDAN-epoxide of 98% and a conversion rate of CDOL to CDON of 84% were achieved.

As can be seen from the above examples, it has surprisingly been found that the epoxidation of the monoethylenically unsaturated organic compound CDEN can be performed in the presence of a second stream comprising primary alcohols, the secondary alcohol CDOL and a derivative comprising a ketone functionality without reducing the conversion of the reaction and likewise the CDON can be obtained in the same reaction with a high conversion rate.

Consequently, it had been shown that the epoxidation (CDEN → CDAN-epoxide) can be performed at high conversion rates, while simultaneously by-products from other reactions can be converted into either useable compounds (CDOL → CDON) or easily separable components, i.e., high boilers (primary alcohols ----> corresponding carboxylic acids). This allows waste streams to be converted in the epoxidation process, eliminating additional purification and conversion steps.

## Claims

1. A method for epoxidizing a monoethylenically unsaturated organic compound, comprising the steps of
(i) mixing a first stream comprising the monoethylenically unsaturated organic compound with up to 25 wt.-% of a second stream comprising a primary alcohol, a secondary alcohol, a polyethylenically unsaturated organic compound or a combination of any of them, wherein wt.-% is based on the total weight of the combined first and second stream;
(ii) oxidizing the mixture obtained in step (i) by at least one peroxide in the presence of a catalyst.

2. The method according to claim 1, further comprising a step (iii) of separating compounds having a boiling point higher than the boiling point of the epoxidized monoethylenically unsaturated organic compound, wherein the compounds preferably are separated by distillation, more preferably by flash-distillation.

3. The method according to claim 2, wherein the distillate obtained in step (iii) comprises the epoxidized monoethylenically unsaturated organic compound and said distillate is optionally used as a reactant in a subsequent reaction without further purification.

4. The method according to claim 3, wherein the subsequent reaction comprises a ring-opening of the epoxide functional group of the epoxidized monoethylenically unsaturated organic compound.

5. The method according to any of claims 3 or 4, wherein the distillate obtained in step (iii) comprises the epoxidized monoethylenically unsaturated organic compound in an amount of at least 50 wt.-%, preferably in an amount of at least 75 wt.-% and further comprises a derivative of the monoethylenically unsaturated organic compound comprising a ketone functionality in an amount of at least 1.0 wt.-%, preferably of at least 2.0 wt.-%, wherein wt.-% is based on the total weight of the distillate.

6. The method according to any of the preceding claims, wherein the second stream is a waste stream.

7. The method according to any of the preceding claims, wherein the monoethylenically unsaturated organic compound is an organic compound having a total of six to twenty carbon atoms, preferably a cyclic organic compound having a total of six to twenty carbon atoms, more preferably a cyclic monoethylenically unsaturated C12 compound, most preferably cyclododecene (CDEN).

8. The method according to any of the preceding claims, wherein the second stream comprises the primary alcohol in an amount of from 0.01 to 20 wt.-%, preferably of from 0.01 to less than 10 wt.-%, wherein wt.-% is based on the total weight of the second stream.

9. The method according to any of the preceding claims, wherein the second stream comprises the secondary alcohol in an amount of from 20 to 99 wt.-%, preferably of from 50 to 99 wt.-%, wherein wt.-% is based on the total weight of the second stream.

10. The method according to any of the preceding claims, wherein the second stream comprises the polyethylenically unsaturated organic compound in an amount of from 0.01 to 20 wt.-%, preferably of from 0.01 to less than 5 wt.-%, wherein wt.-% is based on the total weight of the second stream.

11. The method according to any of the preceding claims, wherein the catalyst is a homogeneous catalyst system.

12. The method according to any of the preceding claims, wherein the method is carried out as a continuous process.

13. A method for synthesizing a lactam, comprising the steps of:
(i) epoxidizing a cyclic monoethylenically unsaturated organic compound to an epoxide;
(ii) rearranging the epoxide to a ketone;
(iii) converting the ketone to an oxime; and
(iv) rearranging the oxime to the lactam;
wherein step (i) comprises the epoxidation according to the method of any of claims 1 to 12.

14. An apparatus for carrying out the method according to any of claims 1 to 12, the apparatus comprising
(i) a first stirred tank reactor or a first series of stirred tank reactors coupled to each other, being adapted to carry out the oxidation of step (ii);
(ii) a second stirred tank reactor or a second series of stirred tank reactors coupled to each other, being adapted to carry out a further reaction of the oxidation product of step (ii) or a subsequent reaction product thereof, thereby producing a waste stream comprising a primary alcohol, a secondary alcohol, a polyethylenically unsaturated organic compound or a combination of any of them; and
(iii) a pipeline connecting the second stirred tank reactor or series of stirred tank reactors with the first stirred tank reactor or series of stirred tank reactors to direct the waste stream produced in the second stirred tank reactor or series of stirred tank reactors to the first stirred tank reactor or series of stirred tank reactors.

15. A monophasic or biphasic composition comprising an organic phase, wherein the organic phase comprises an epoxidized monoethylenically unsaturated organic compound in an amount of from 50 to 99 wt.-%, preferably of from 75 to 98 wt.-%, a derivative of the monoethylenically unsaturated organic compound comprising a ketone functionality in an amount of from 1.0 to 20 wt.-%, preferably of from 2.0 to 20 wt.-%, and tungsten in an amount of from 500 to 5000 ppm, preferably of from 1000 to 3000 ppm; wherein wt.-% and ppm are based on the total weight of the organic phase.
